# EUROPEAN PATENT APPLICATION

(11) **EP 4 410 994 A1**
(43) Date of publication of application: **07.08.2024**
(21) Application number: 22876206.8
(22) Date of filing: 27.09.2022
(51) Int. Cl.: C12Q 1/6844, C08F 130/02, C08F 230/02

(54) **NUCLEIC ACID AMPLIFICATION PROMOTER AND TEST METHOD USING SAME**

(30) Priority: 30.09.2021 JP 2021161241
(71) Applicant: NOF Corporation, Shibuya-ku Tokyo 150-6019 (JP)
(72) Inventor: SUZUKI, Hirotaka, Kawasaki-shi, Kanagawa 210-0865 (JP); MATSUDA, Masaru, Kawasaki-shi, Kanagawa 210-0865 (JP)
(74) Representative: Vossius & Partner Patentanwälte Rechtsanwälte mbB
(86) International application number: PCT/JP2022/035891
(87) International publication number: WO 2023/054340

(57) **Abstract**

The present invention provides a nucleic acid amplification-promoting agent consisting of a polymer containing a constitutional unit derived from a monomer having a phosphorylcholine group.

## Description

### [Technical Field]

The present invention relates to a nucleic acid amplification-promoting agent and test methods using same.

### [Background Art]

A nucleic acid amplification method is a method for amplifying the target nucleic acid from several copies to tens of thousands of times more copies, and is actively used in various areas for gene cloning and genetic testing.

A representative method of nucleic acid amplification method is the Polymerase Chain Reaction (PCR) method. In a typical PCR method, nucleic acid is amplified by repeating the three steps of (1) denaturation of template DNA (separation from double-stranded DNA to single-stranded DNA), (2) annealing of primer to single-stranded template DNA, and (3) elongation of primer by DNA polymerase.

A typical PCR method was originally a method for amplifying DNA. It can also be applied to the amplification of RNA, and such PCR method is called a reverse transcription polymerase chain reaction method (hereinafter sometimes to be abbreviated as "RT-PCR method"). In the RT-PCR method, complementary DNA (hereinafter sometimes to be abbreviated as "cDNA") is synthesized from RNA by an enzymatic reaction using reverse transcriptase, and PCR is performed using this cDNA as a template to amplify RNA. The RT-PCR method is further divided into a 2-step method in which the reverse transcription reaction and the PCR method are performed in separate containers, and a 1-step method in which these are performed as a series of reactions in the same container.

A method for determining the amount of initial nucleic acid based on the amount of amplified product obtained by the PCR method is also known, and such PCR method is called a quantitative polymerase chain reaction method (hereinafter sometimes to be abbreviated as "qPCR method").

Furthermore, a reverse transcription-quantitative PCR method (hereinafter sometimes to be abbreviated as "RT-qPCR method"), which is a combination of the RT-PCR method and the qPCR method, is also known.

In recent years, moreover, a method called a digital PCR method (hereinafter sometimes to be abbreviated as "dPCR method") has also been developed in which a reaction liquid is dispensed to some tens to tens of thousands of extremely small compartments by applying microchannel formation technology or the like, and PCRs are performed all at once, and the concentration of initial nucleic acid is determined using a statistical model from the proportion of the compartments with amplification.

In addition to the purpose of determining the presence or absence and/or the amount of the target nucleic acid as described above, the PCR method is also used for the purpose of determining the base sequence of the target nucleic acid (sequencing). The sequencing PCR method includes various methods represented by the Sanger method, and in many of them, the basic principle is the end-point PCR method.

When using nucleic acid amplification methods, it is often a problem to increase the concentration of amplification products in any of when the amplification product itself is the target product, when the purpose is to qualitatively or quantitatively measure the initial amount of nucleic acid, and when the purpose is to determine the base sequence of a nucleic acid. Therefore, various techniques for promoting nucleic acid amplification in PCR have been investigated.

For example, it is widely practiced to try to promote nucleic acid amplification by adding salts (e.g., potassium chloride, ammonium sulfate, etc.), betaine, polyhydric alcohols, polyethylene glycol, and the like to a reaction liquid in the PCR method. However, there is a limit to the promotion of nucleic acid amplification by the addition of these components, and a higher level has been demanded.

As other method, for example, a method of destabilizing double-stranded DNA by adding a single-stranded DNA-binding protein (SSB) is known. Patent Literature 1 discloses a mutant PCNA (proliferating nuclear antigen) monomer as a highly versatile DNA replication-promoting factor (additive) for promoting the elongation reaction of DNA. However, these additives composed of proteins have problems such as difficulty in large-scale production, problems in storage stability, and high production costs.

### [Citation List]

### [Patent Literature]

[Patent Literature 1]
WO 2007/004654

### [Summary of Invention]

### [Technical Problem]

The present invention aims to provide a promoting agent for nucleic acid amplification which is superior in mass productivity and preservation stability as compared with additives composed of proteins.

### [Solution to Problem]

[1] A nucleic acid amplification-promoting agent consisting of a polymer comprising a constitutional unit derived from a monomer having a phosphorylcholine group.
[2] The nucleic acid amplification-promoting agent of the aforementioned [1], wherein the aforementioned polymer comprises one or more polymers selected from the following group a:
   [group a]
   (a-1) a polymer comprising a constitutional unit derived from 2-(meth)acryloyloxyethylphosphorylcholine alone,
   (a-2) a copolymer comprising a constitutional unit derived from 2-(meth)acryloyloxyethylphosphorylcholine and a constitutional unit derived from (meth)acrylic acid,
   (a-3) a copolymer comprising a constitutional unit derived from 2-(meth)acryloyloxyethylphosphorylcholine and a constitutional unit derived from a C₂-C₂₀ alkyl (meth)acrylate,
   (a-4) a copolymer comprising a constitutional unit derived from 2-(meth)acryloyloxyethylphosphorylcholine, a constitutional unit derived from a C₂-C₈ alkyl (meth)acrylate, and a constitutional unit derived from a C₂-C₈ alkyl (meth)acrylate having two or more hydroxy groups as substituents, and
   (a-5) a copolymer comprising a constitutional unit derived from 2-(meth)acryloyloxyethylphosphorylcholine and a constitutional unit derived from a polyethylene glycol (meth)acrylate.
[3] The nucleic acid amplification-promoting agent of the aforementioned [2], wherein the 2-(meth)acryloyloxyethylphosphorylcholine is 2-methacryloyloxyethylphosphorylcholine.
[4] The nucleic acid amplification-promoting agent of the aforementioned [2] or [3], wherein the (meth)acrylic acid is methacrylic acid.
[5] The nucleic acid amplification-promoting agent of any one of the aforementioned [2] to [4], wherein the C₂-C₂₀ alkyl (meth)acrylate is ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, lauryl (meth)acrylate, tridecyl (meth)acrylate, myristyl (meth)acrylate, pentadecyl (meth)acrylate, cetyl (meth)acrylate, margaryl (meth)acrylate, stearyl (meth)acrylate, nonadecyl (meth)acrylate, or arachidyl (meth)acrylate, preferably propyl (meth)acrylate, butyl (meth)acrylate, pentyl (meth)acrylate, lauryl (meth)acrylate, myristyl (meth)acrylate, cetyl (meth)acrylate, or stearyl (meth)acrylate, more preferably butyl (meth)acrylate or stearyl (meth)acrylate, further preferably butyl methacrylate or stearyl methacrylate.
[6] The nucleic acid amplification-promoting agent of any one of the aforementioned [2] to [5], wherein the C₂-C₈ alkyl (meth)acrylate (specifically C₂-C₉ alkyl (meth)acrylate without a hydroxy group) is ethyl (meth)acrylate, propyl (meth)acrylate, butyl (meth)acrylate, pentyl (meth)acrylate, hexyl (meth)acrylate, heptyl (meth)acrylate, or octyl (meth)acrylate, preferably propyl (meth)acrylate, butyl (meth)acrylate, or pentyl (meth)acrylate, more preferably propyl methacrylate, butyl methacrylate, or pentyl methacrylate, further preferably butyl methacrylate.
[7] The nucleic acid amplification-promoting agent of any one of the aforementioned [2] to [6], wherein the C₂-C₈ alkyl (meth)acrylate having two or more hydroxy groups as substituents is glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, or sorbitol mono(meth)acrylate, preferably glycerol mono(meth)acrylate or xylitol mono(meth)acrylate, more preferably glycerol mono(meth)acrylate, further preferably glycerol monomethacrylate.
[8] The nucleic acid amplification-promoting agent of any one of the aforementioned [2] to [7], wherein the polyethylene glycol (meth)acrylate is a polyethylene glycol mono(meth)acrylate, a methoxypolyethylene glycol (meth)acrylate, or an ethoxypolyethylene glycol (meth)acrylate, preferably a methoxypolyethylene glycol (meth)acrylate or an ethoxypolyethylene glycol (meth)acrylate, more preferably a methoxypolyethylene glycol methacrylate or an ethoxypolyethylene glycol methacrylate, further preferably a methoxypolyethylene glycol methacrylate.
[9] A test method using the nucleic acid amplification-promoting agent of any one of the aforementioned [1] to [8].

### [Advantageous Effects of Invention]

The nucleic acid amplification-promoting agent of the present invention can increase the amount of nucleic acid amplification products obtained by the nucleic acid amplification method. Therefore, the nucleic acid amplification-promoting agent of the present invention can be used, for example, to improve the yield of a desired nucleic acid by a nucleic acid amplification method, to improve the performance of tests by a nucleic acid amplification method, to improve the performance of sequencing by a nucleic acid amplification method, and the like. In addition, since the nucleic acid amplification-promoting agent of the present invention is a synthetic polymer, it is superior in the mass productivity and preservation stability as compared with additives composed of proteins.

### [Description of Embodiments]

The present invention is described in detail below.

In the present specification, the "(meth)acryloyloxy group" basically means an "acryloyloxy group or methacryloyloxy group". When a plurality of the (meth)acryloyloxy groups may be present, the "(meth)acryloyloxy group" means an "acryloyloxy group and/or methacryloyloxy group". Other terms similar to the "(meth)acryloyloxy group" also mean the same as the "(meth)acryloyloxy group".

When stepwise numerical ranges are described in the present specification, the lower limit and the upper limit of each numerical range can be combined. In the case of, for example, "preferably 10 to 100, more preferably 20 to 90", the preferred lower limit 10 can be combined with the more preferred upper limit 90 (i.e., the numerical range of "10 to 90" is also within the range of the present specification).

In the present specification, "Cₓ-C_{y}" (x, y: integer) means that the "carbon number is x to y".

### [Nucleic acid amplification-promoting agent]

The nucleic acid amplification-promoting agent of the present invention consists of a polymer containing a constitutional unit derived from a monomer having a phosphorylcholine group (hereinafter sometimes to be referred to as "the polymer of the present invention"). As used herein, the nucleic acid amplification-promoting agent means an additive that increases the amount of an amplification product in a nucleic acid amplification method.

Examples of the nucleic acid amplification method in which the nucleic acid amplification-promoting agent of the present invention can be used include Polymerase Chain Reaction (PCR) method, Loop mediated isothermal Amplification (LAMP) method, Transcription Mediated Amplification (TMA) method, Isothermal and Chimeric primer-initiated Amplification of Nucleic acids (ICAN) method, Strand Displacement Amplification (SDA) method, Ligase Chain Reaction (LCR) method, Nucleic Acid Seqence-Based Amplification (NASBA) method, and the like. The nucleic acid amplification method is preferably a PCR method. That is, the nucleic acid amplification-promoting agent of the present invention is preferably used in the PCR method.

As the PCR method, an appropriate method may be selected depending on the kind of template nucleic acid, that is, the nucleic acid to be amplified. For example, when the template nucleic acid is a double-stranded DNA, a typical PCR method is preferably selected, and when the template nucleic acid is a single-stranded RNA, RT-PCR method is preferably selected.

Furthermore, in medical tests, the qPCR method is preferably selected. That is, the qPCR method is preferably selected for tests that target DNA such as bacterial tests, fungal tests, DNA virus tests, and the like, and the RT-qPCR method is preferably selected for tests that target RNA such as RNA virus tests and the like.

Only one kind of the nucleic acid amplification-promoting agent of the present invention may be used, or two or more kinds thereof may be used in combination. In addition, the nucleic acid amplification-promoting agent of the present invention may be used in combination with other additives as long as the effect of the present invention is not impaired.

The polymer of the present invention contains a constitutional unit derived from a monomer having a phosphorylcholine group represented by the following formula:

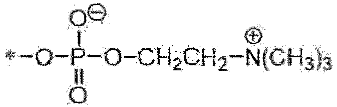

wherein * is a bonding position
(hereinafter sometimes to be abbreviated as "phosphorylcholine group-containing unit").

Preferred examples of the monomer having a phosphorylcholine group include 2-(meth)acryloyloxyethylphosphorylcholine, 2-(meth)acrylamidoethylphosphorylcholine, alkylphosphorylcholine allyl ether, alkylphosphorylcholine vinyl ether, and the like. From the aspects of the preservation stability of the polymer and the availability of the starting material, 2-methacryloyloxyethylphosphorylcholine and 2-methacrylamidoethylphosphorylcholine are more preferred, and 2-methacryloyloxyethylphosphorylcholine is more preferred.

The proportion of the phosphorylcholine group-containing unit in the whole constitutional units of the polymer of the present invention is preferably 30 to 100 mol%, more preferably 70 to 100 mol%, further preferably 80 to 100 mol%.

While the weight average molecular weight of the polymer of the present invention is not particularly limited, it is preferably 5,000 to 2,000,000, more preferably 10,000 to 1,500,000. The weight average molecular weight can be determined based on polyethylene glycol by gel filtration chromatography using, for example, EcoSEC system (manufactured by Tosoh Corporation) or the like.

The polymer of the present invention preferably contains one or more polymers selected from the following group a:
[group a]
(a-1) a polymer containing a constitutional unit derived from 2-(meth)acryloyloxyethylphosphorylcholine alone (i.e., polymer consisting of the aforementioned constitutional units) (hereinafter sometimes to be abbreviated as "polymer (a-1)"),
(a-2) a copolymer containing a constitutional unit derived from 2-(meth)acryloyloxyethylphosphorylcholine and a constitutional unit derived from (meth)acrylic acid (hereinafter sometimes to be abbreviated as "polymer (a-2)"),
(a-3) a copolymer containing a constitutional unit derived from 2-(meth)acryloyloxyethylphosphorylcholine and a constitutional unit derived from a C₂-C₂₀ alkyl (meth)acrylate (hereinafter sometimes to be abbreviated as "polymer (a-3)"),
(a-4) a copolymer containing a constitutional unit derived from 2-(meth)acryloyloxyethylphosphorylcholine, a constitutional unit derived from a C₂-C₈ alkyl (meth) acrylate, and a constitutional unit derived from a C₂-C₈ alkyl (meth) acrylate having two or more hydroxy groups as substituents (hereinafter sometimes to be abbreviated as "polymer (a-4)"), and
(a-5) a copolymer containing a constitutional unit derived from 2-(meth)acryloyloxyethylphosphorylcholine and a constitutional unit derived from a polyethylene glycol (meth)acrylate (hereinafter sometimes to be abbreviated as "polymer (a-5)").

When the aforementioned polymer is a copolymer, the copolymer may be a random copolymer, an alternating copolymer, a block copolymer, a graft polymer, or a copolymer containing two or more structures thereof. It is preferably a random copolymer from the aspect of the manufacturability of the polymer.

The polymer (a-1) may be homopolymer of (meth)acryloyloxyethylphosphorylcholine, or copolymer of acryloyloxyethylphosphorylcholine and methacryloyloxyethylphosphorylcholine. The polymer (a-1) is preferably a homopolymer of 2-(meth)acryloyloxyethylphosphorylcholine, more preferably a homopolymer of 2-methacryloyloxyethylphosphorylcholine.

While the weight average molecular weight of polymer (a-1) is not particularly limited, it is preferably 20,000 to 2,000,000, more preferably 100,000 to 1,500,000, further preferably 500,000 to 1,500,000.

For the formation of polymer (a-2), only one kind of the (meth)acryloyloxyethylphosphorylcholine (i.e., acryloyloxyethylphosphorylcholine or methacryloyloxyethylphosphorylcholine) may be used, or two kinds thereof (i.e., acryloyloxyethylphosphorylcholine and methacryloyloxyethylphosphorylcholine) may be used. As (meth)acryloyloxyethylphosphorylcholine for the formation of polymer (a-2), methacryloyloxyethylphosphorylcholine is preferred from the aspects of the preservation stability of the polymer and the availability of the starting material.

For the formation of polymer (a-2), only one kind of the (meth)acrylic acid (hereinafter sometimes to be abbreviated as "MAc") (i.e., acrylic acid or methacrylic acid) may be used, or two kinds thereof (i.e., acrylic acid and methacrylic acid) may be used. As MAc, methacrylic acid is preferred from the aspect of the preservation stability of the polymer.

The proportion of the constitutional unit derived from MAc in the whole constitutional units of polymer (a-2) is preferably 1 to 70 mol%, more preferably 60 to 80 mol%. The rest of the constitutional units of polymer (a-2) are preferably constitutional units derived from (meth)acryloyloxyethylphosphorylcholine.

While the weight average molecular weight of polymer (a-2) is not particularly limited, it is preferably 10,000 to 2,000,000, more preferably 100,000 to 1,500,000, further preferably 500,000 to 1,000,000.

The polymer (a-2) is
preferably a copolymer of 2-(meth)acryloyloxyethylphosphorylcholine and (meth)acrylic acid,
more preferably a copolymer of 2-methacryloyloxyethylphosphorylcholine and methacrylic acid.

For the formation of polymer (a-3), only one kind of the (meth)acryloyloxyethylphosphorylcholine may be used, or two kinds thereof may be uses. As (meth)acryloyloxyethylphosphorylcholine for the formation of polymer (a-3), methacryloyloxyethylphosphorylcholine is preferred from the aspects of the preservation stability of the polymer and the availability of the starting material.

For the formation of polymer (a-3), only one kind of the C₂-C₂₀ alkyl (meth)acrylate (hereinafter sometimes to be abbreviated as "C₂-C₂₀ AMA") may be used, or two or more kinds thereof may be used in combination. Examples of the C₂-C₂₀ AMA include ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, lauryl (meth)acrylate, tridecyl (meth)acrylate, myristyl (meth)acrylate, pentadecyl (meth)acrylate, cetyl (meth)acrylate, margaryl (meth)acrylate, stearyl (meth)acrylate, nonadecyl (meth)acrylate, arachidyl (meth)acrylate, and monomers in which these alkyl group moieties are replaced with the corresponding structural isomers. Among these, propyl (meth)acrylate, butyl (meth)acrylate, pentyl (meth)acrylate, lauryl (meth)acrylate, myristyl (meth)acrylate, cetyl (meth)acrylate, and stearyl (meth)acrylate are preferred, butyl (meth)acrylate and stearyl (meth)acrylate are more preferred, and butyl methacrylate and stearyl methacrylate are more preferred.

The proportion of the constitutional unit derived from C₂-C₂₀ AMA in the whole constitutional units of polymer (a-3) is preferably 5 to 80 mol%, more preferably 10 to 30 mol%. The rest of the constitutional units of the polymer (a-3) are preferably constitutional units derived from (meth)acryloyloxyethylphosphorylcholine.

While the weight average molecular weight of polymer (a-3) is not particularly limited, it is preferably 5,000 to 2,000,000, more preferably 10,000 to 1,000,000, further preferably 20,000 to 1,000,000.

The polymer (a-3) is
preferably a copolymer of 2-(meth)acryloyloxyethylphosphorylcholine, and ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, lauryl (meth)acrylate, tridecyl (meth)acrylate, myristyl (meth)acrylate, pentadecyl (meth)acrylate, cetyl (meth)acrylate, margaryl (meth)acrylate, stearyl (meth)acrylate, nonadecyl (meth)acrylate, or arachidyl (meth)acrylate,
more preferably a copolymer of 2-(meth)acryloyloxyethylphosphorylcholine, and propyl (meth)acrylate, butyl (meth)acrylate, pentyl (meth)acrylate, lauryl (meth)acrylate, myristyl (meth)acrylate, cetyl (meth)acrylate, or stearyl (meth)acrylate,
further preferably a copolymer of 2-(meth)acryloyloxyethylphosphorylcholine, and butyl (meth)acrylate or stearyl (meth)acrylate,
particularly preferably a copolymer of 2-methacryloyloxyethylphosphorylcholine, and butyl methacrylate or stearyl methacrylate.

For the formation of polymer (a-4), only one kind of the (meth)acryloyloxyethylphosphorylcholine may be used, or two kinds thereof may be used. As (meth)acryloyloxyethylphosphorylcholine for the formation of polymer (a-4), methacryloyloxyethylphosphorylcholine is preferred from the aspects of the preservation stability of the polymer and the availability of the starting material.

For the formation of polymer (a-4), only one kind of the C₂-C₈ alkyl (meth)acrylate (specifically C₂-C₈ alkyl (meth)acrylatewithout a hydroxy group, hereinafter sometimes to be abbreviated as "C₂-C₈ AMA") may be used, or two or more kinds thereof may be used in combination. Examples of the C₂-C_{B} AMA include ethyl (meth)acrylate, propyl (meth)acrylate, butyl (meth)acrylate, pentyl (meth)acrylate, hexyl (meth)acrylate, heptyl (meth)acrylate, octyl (meth)acrylate, monomers in which these alkyl group moieties are replaced with the corresponding structural isomers, and the like. From the aspect of high nucleic acid amplification-promoting effect, propyl (meth)acrylate, butyl (meth)acrylate, and pentyl (meth)acrylate are preferred. From the aspect of the preservation stability of the polymer, propyl methacrylate, butyl methacrylate, and pentyl methacrylate are more preferred, and butyl methacrylate is more preferred.

For the formation of polymer (a-4), only one kind of the C₂-C₈ alkyl (meth)acrylate having two or more hydroxy groups as substituents (hereinafter sometimes to be abbreviated as "HO-AMA") may be used, or two or more kinds thereof may be used in combination. Examples of the HO-AMA include glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, sorbitol mono(meth)acrylate, and the like. Among these, glycerol mono(meth)acrylate and xylitol mono(meth)acrylate are preferred, glycerol mono(meth)acrylate is more preferred, and glycerol monomethacrylate is further preferred.

The proportion of the constitutional unit derived from C₂-C₈ AMA in the whole constitutional units of polymer (a-4) is preferably 10 to 60 mol%, more preferably 20 to 60 mol%, further preferably 30 to 60 mol%. The proportion of the constitutional unit derived from HO-AMA in the whole constitutional units of polymer (a-4) is preferably 10 to 60 mol%, more preferably 10 to 50 mol%, further preferably 10 to 40 mol%. The rest of the constitutional units of the polymer (a-4) are preferably constitutional units derived from (meth)acryloyloxyethylphosphorylcholine.

The "C₂-C₈ alkyl (meth)acrylate" for forming polymer (a-4) is included in the "C₂-C₂₀ alkyl (meth)acrylate" for forming polymer (a-3). In the present invention, among the copolymers containing a constitutional unit derived from 2-(meth)acryloyloxyethylphosphorylcholine and a constitutional unit derived from a C₂-C₈ alkyl (meth) acrylate, a copolymer further containing a constitutional unit derived from a C₂-C₈ alkyl (meth)acrylate having two or more hydroxy groups as substituents is classified as polymer (a-4), and a copolymer that does not contain the aforementioned constitutional unit is classified as polymer (a-3).

While the weight average molecular weight of polymer (a-4) is not particularly limited, it is preferably 10,000 to 500,000, more preferably 10,000 to 100,000, further preferably 10,000 to 50,000.

The polymer (a-4) is
preferably a copolymer of 2-(meth)acryloyloxyethylphosphorylcholine, and ethyl (meth)acrylate, propyl (meth)acrylate, butyl (meth)acrylate, pentyl (meth)acrylate, hexyl (meth)acrylate, heptyl (meth)acrylate, or octyl (meth)acrylate, and glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, or sorbitol mono(meth)acrylate,
more preferably a copolymer of 2-(meth)acryloyloxyethylphosphorylcholine, and propyl (meth)acrylate, butyl (meth)acrylate, or pentyl (meth)acrylate, and glycerol mono(meth)acrylate or xylitol mono(meth)acrylate,
further preferably a copolymer of 2-methacryloyloxyethylphosphorylcholine, and propyl methacrylate, butyl methacrylate, or pentyl methacrylate, and glycerol monomethacrylate,
particularly preferably a copolymer of 2-methacryloyloxyethylphosphorylcholine, butyl methacrylate, and glycerol monomethacrylate.

For the formation of polymer (a-5), only one kind of the (meth)acryloyloxyethylphosphorylcholine may be used, or two kinds thereof may be used. As (meth)acryloyloxyethylphosphorylcholine for the formation of polymer (a-5), methacryloyloxyethylphosphorylcholine is preferred from the aspects of the preservation stability of the polymer and the availability of the starting material.

For the formation of polymer (a-5), only one kind of the polyethylene glycol (meth)acrylate (hereinafter sometimes to be abbreviated as "PEG-MA") may be used, or two or more kinds thereof may be used in combination. Examples of the PEG-MA include polyethylene glycol mono(meth)acrylate, methoxypolyethylene glycol (meth)acrylate, ethoxypolyethylene glycol (meth) acrylate, and the like. From the aspect of the preservation stability of the polymer, a methoxypolyethylene glycol (meth)acrylate, an ethoxypolyethylene glycol (meth)acrylate is preferred, a methoxypolyethylene glycol methacrylate, and an ethoxypolyethylene glycol methacrylate are more preferred, and a methoxypolyethylene glycol methacrylate is more preferred.

The molecular weight or number average molecular weight of the polyethylene glycol chain in the polymer (a-5) is preferably 50 to 10,000, more preferably 50 to 5,000, further preferably 50 to 1,000, and particularly preferably 100 to 1,000, from the aspects of the availability of the starting material and the nucleic acid amplification-promoting effect. The molecular weight or number average molecular weight of the polyethylene glycol chain in polymer (a-5) can be calculated from, for example, the molecular weight or number average molecular weight of PEG-MA used to form polymer (a-5). In addition, the molecular weight of PEG-MA can be determined, for example, based on the hydroxyl value calculated by the method described in JIS K 1557. In addition, the number average molecular weight of PEG-MA can be determined in terms of polyethylene glycol by, for example, gel filtration chromatography.

The proportion of the constitutional unit derived from PEG-MA in the whole constitutional units of the polymer (a-5) is preferably 5 to 40 mol%, more preferably 5 to 20 mol%. The rest of the constitutional units of the polymer (a-5) are preferably constitutional units derived from (meth)acryloyloxyethylphosphorylcholine.

While the weight average molecular weight of polymer (a-5) is not particularly limited, it is preferably 100,000 to 1,000,000, more preferably 200,000 to 1,000,000, further preferably 300,000 to 800,000.

The polymer (a-5) is
preferably a copolymer of 2-(meth)acryloyloxyethylphosphorylcholine, and a polyethylene glycol mono(meth)acrylate, a methoxypolyethylene glycol (meth)acrylate, or an ethoxypolyethylene glycol (meth)acrylate,
more preferably a copolymer of 2-(meth)acryloyloxyethylphosphorylcholine, and a methoxypolyethylene glycol (meth)acrylate or an ethoxypolyethylene glycol (meth)acrylate,
further preferably a copolymer of 2-a methacryloyloxyethylphosphorylcholine, a and methoxypolyethylene glycol methacrylate or an ethoxypolyethylene glycol methacrylate,
particularly preferably a copolymer of 2-methacryloyloxyethylphosphorylcholine and a methoxypolyethylene glycol methacrylate.

The polymer of the present invention may contain, as long as the effect of the present invention is not impaired, a constitutional unit derived from a monomer other than the aforementioned monomer (hereinafter to be indicated as "other monomer"). Examples of other monomer include benzyl (meth)acrylate, isobornyl (meth)acrylate, and the like. The proportion of a constitutional unit derived from other monomer in the whole constitutional units of the polymer of the present invention is preferably not more than 20 mol%, more preferably not more than 10 mol%. More preferably, the polymer of the present invention does not contain a constitutional unit derived from other monomer.

As each monomer for the preparation of the polymer of the present invention, a commercially available product may be used, or the monomer may be produced by a known method. The polymer of the present invention can be produced by known methods (e.g., the method described in WO 2018/216628).

The nucleic acid amplification-promoting agent of the present invention can be easily used as a nucleic acid amplification-promoting agent by being added to a reaction composition of a desired nucleic acid amplification reaction before the start of the reaction. The amount of the nucleic acid amplification-promoting agent of the present invention to be added to the reaction composition in the aforementioned nucleic acid amplification reaction is preferably 0.001 to 5 w/v%, more preferably 0.01 to 1 w/v%, further preferably 0.01 to 0.1 w/v%.

### [Test method]

The present invention further provides a test method using the nucleic acid amplification-promoting agent of the present invention.

Examples of the preferred embodiment of the aforementioned test method include a method including amplifying the nucleic acid of a test target (sample) by a nucleic acid amplification method using a reaction composition containing a test target (sample) and the nucleic acid amplification-promoting agent of the present invention, and determining the presence or absence or concentration of the test target from the amount of the amplification reaction product. The nucleic acid amplification method to be used in the test method of the present invention is as described above.

The aforementioned reaction composition is preferably a composition (more preferably mixed solution) containing the nucleic acid amplification-promoting agent of the present invention, a reaction reagent, and a test target (sample), and can be prepared according to known nucleic acid amplification methods represented by the PCR method.

Examples of the aforementioned reaction reagent include buffer, substrate, primer, DNA polymerase, fluorescent DNA staining reagent, fluorescent probe, passive reference, nucleic acid, and the like.

The aforementioned buffer is not particularly limited. Examples thereof include buffers with a pH adjusted to 6 to 9, more preferably about 7 to 8, by mixing a base such as tris(hydroxymethyl)aminomethane, Tricine, Bicine, or the like and an acid such as sulfuric acid, hydrochloric acid, acetic acid, phosphoric acid, or the like. The buffer desirably contains a magnesium salt and/or a manganese salt as appropriate. The buffer may further contain a salt such as potassium chloride, ammonium sulfate, or the like. The buffer may further contain a water-soluble organic solvent such as dimethyl sulfoxide, dimethylformamide, formamide, glycerol, or the like. The buffer may further contain a surfactant such as polyoxysorbitan fatty acid ester, polyoxyethylene alkylphenyl ether, or the like. The buffer may further contain a protein such as bovine serum albumin or the like. The buffer may further contain a water-soluble polymer such as polyethylene glycol or the like.

While the aforementioned substrate is not particularly limited, examples thereof include a mixture (dNTPs) of deoxyadenosine triphosphate (dATP), deoxythymidine triphosphate (dTTP), deoxyguanosine triphosphate (dGTP), and deoxycytidine triphosphate (dCTP). It is also possible to partially and/or entirely substitute dTTP with deoxyuridine triphosphate (dUTP). In sequencing PCR and the like, an appropriate amount of a mixture of dideoxyadenosine triphosphate (ddATP), dideoxythymidine triphosphate (ddTTP), dideoxyguanosine triphosphate (ddGTP), and dideoxycytidine triphosphate (ddCTP), or a fluorescently labeled product thereof may also be added.

The aforementioned primer is not particularly limited. Examples thereof include oligonucleotides with a length of about 15 to 30 bases designed and prepared by known methods. The primers may be subjected to, as appropriate, fluorescent labeling with fluorescein or the like, or isotope labeling with a heavy element, or the like. Only one kind of the primer may be used, or two kinds of primers may be used as one pair, and primers in a plurality of pairs may also be used.

As the aforementioned DNA polymerase, known DNA polymerase can be used. From the aspect of heat resistance, enzymes derived from thermophilic bacteria, thermophilic archaea, hyperthermophile, or hyperthermophile archaea, and mutant enzymes thereof are preferred. One or more DNA polymerases is/are appropriately selected from a DNA-dependent DNA polymerase, an RNA-dependent DNA polymerase (reverse transcriptase), or an enzyme having both functions, depending on the purpose of nucleic acid amplification. Moreover, whether to use a DNA polymerase having nuclease activity or a DNA polymerase having no nuclease activity is appropriately determined.

The aforementioned fluorescent DNA staining reagent is not particularly limited, and examples thereof include SYBR^{™} Green I and the like.

The aforementioned fluorescent probe is not particularly limited, and examples thereof include TaqMan^{™} probe.

The aforementioned passive reference may be selected appropriately according to the purpose of nucleic acid amplification. Examples of the passive reference include ROX^{™} Dye and the like.

As the aforementioned nucleic acid, any DNA and/or RNA may be used, for example, as an exogenous control gene, in addition to the aforementioned primers and fluorescent probes. Here, the nucleic acid may be synthesized in vitro, or may be prepared from cells, microorganisms, viruses, or the like by known methods. Here, the cells, microorganisms, viruses, and the like may be those collected from human, animals, or plants in the natural world or environment, or may be those obtained by isolation and culture.

The aforementioned reaction reagent may further contain oil such as mineral oil or the like.

In addition, a solid support such as silica bead, magnetic bead, or the like may be further added to the aforementioned reaction reagent.

It is also preferably performed to select a plurality of the above-mentioned respective components and mix them in advance to give a master-mix (sometimes called primer mix, premix, etc.).

The aforementioned sample is a test target. Examples of the sample include virus, bacterial cell, cell, body fluid, tissue, and the like of test targets, suspensions thereof, nucleic acid extracts prepared from these, nucleic acid standard solutions, blank test samples, and the like. The aforementioned sample can be appropriately selected according to the mode of tests.

Examples of the aforementioned test include medical tests, veterinary tests, forensic tests, medicine tests, food inspections, environmental audits, and the like. The test method of the present invention is preferably used for medical tests.

It is also preferable to combine the respective components of the reaction composition for the aforementioned nucleic acid amplification reaction with members such as a reaction container and a sample collection device and the like to form a test kit. The aforementioned test kit is preferably a pharmaceutical product for in vitro diagnosis.

The determination of the presence or absence or concentration of the test target based on the amount of the aforementioned amplification reaction product can be performed using known nucleic acid detection methods. As the detection method, for example, a method including purifying an amplification reaction product and measuring the turbidity in the ultraviolet region of the purified nucleic acid solution can be mentioned. As another method, for example, a method including developing a reaction product by gel electrophoresis, staining the nucleic acid with a nucleic acid staining reagent such as ethidium bromide or the like, and qualifying or quantifying the staining intensity thereof by visual observation or by instrumental analysis can be mentioned. As still another method, for example, a method including adding an intercalating reagent such as CYBR Green I or the like to a reaction composition of the nucleic acid amplification reaction and monitoring the optical property of the intercalating reagent can be mentioned.

### [Nucleic acid amplification method]

The present invention also provides a method for amplifying the nucleic acid of a sample by a nucleic acid amplification method using a reaction composition containing a sample and the nucleic acid amplification-promoting agent of the present invention (hereinafter referred to as "the method of the present invention"). The nucleic acid amplification-promoting agent and nucleic acid amplification method used in the method of the present invention are as described above.

The reaction composition used in the method of the present invention is preferably a composition (more preferably a mixed solution) containing the nucleic acid amplification-promoting agent of the present invention, a reaction reagent, and a sample, and can be prepared according to known nucleic acid amplification methods represented by the PCR method. The reaction reagents used in the method of the present invention are described above.

### [Use]

The present invention also provides the use of the nucleic acid amplification-promoting agent of the present invention for amplifying the nucleic acid of a sample by a nucleic acid amplification method (hereinafter referred to as "the use of the present invention"). The nucleic acid amplification-promoting agent and nucleic acid amplification method for use in the present invention are as described above.

### [Example]

The present invention is explained in more detail in the following by referring to Examples and the like, which are not to be construed as limitative.

### [Synthesis of polymers (nucleic acid amplification-promoting agents)]

The polymers of the present invention were prepared as follows.

### [Synthetic Example 1]

Polymer 1 corresponding to polymer (a-1) was prepared as follows.

2-Methacryloyloxyethylphosphorylcholine (hereinafter sometimes to be indicated as "MPC") (40.0 g) was weighed into a glass flask for polymerization, purified water (60.0 g) was added to dissolve same, and azobisisobutyronitrile (hereinafter to be referred to as "AIBN") (0.31 g) was added as a polymerization initiator to the obtained solution. After the inside of the reaction container was sufficiently replaced with nitrogen, polymerization was performed by heating at 70°C for 6 hr while stirring. The obtained reaction solution was ice-cooled and added dropwise to diethyl ether to precipitate a polymer. The precipitate was collected by filtration, washed with diethyl ether, and vacuum dried to give a homopolymer (hereinafter to be indicated as "polymer 1" in the present specification) as a white powder. The weight average molecular weight of polymer 1 was 1,030,000 based on polyethylene glycol as measured by gel filtration chromatography (hereinafter sometimes to be abbreviated as "GPC") under the below-mentioned conditions.

### [Synthetic Example 2]

Polymer 2 corresponding to polymer (a-2) was prepared as follows.

MPC (6.0 g) and methacrylic acid (4.0 g) as MAc (MPC/MAc=30/70 (molar ratio)) were weighed into a glass flask for polymerization, purified water (90.0 g) was added to dissolve same, and AIBN (0.78 g) was added to the obtained solution. After the inside of the reaction container was sufficiently replaced with nitrogen, polymerization was performed by heating at 60°C for 5 hr while stirring. Thereafter, in the same manner as in Synthetic Example 1, a random copolymer (hereinafter to be indicated as "polymer 2" in the present specification) was obtained. The weight average molecular weight of polymer 2 was 680,000 based on polyethylene glycol as measured by GPC under the below-mentioned conditions.

### [Synthetic Example 3-1]

Polymer 3-1 corresponding to polymer (a-3) was prepared as follows.

MPC (19.4 g) and n-butyl methacrylate (2.2 g) as C₂-C₂₀ AMA (MPC/C₂-C₂₀ AMA=80/20 (molar ratio)) were weighed into a glass flask for polymerization, purified water (39.3 g) and ethanol (39.3 g) were added to dissolve same, and AIBN (0.02 g) was added to the obtained solution. Thereafter, in the same manner as in Synthetic Example 1, a random copolymer (hereinafter to be indicated as "polymer 3-1" in the present specification) was obtained. The weight average molecular weight of polymer 3-1 was 600,000 based on polyethylene glycol as measured by GPC under the below-mentioned conditions.

### [Synthetic Example 3-2]

Polymer 3-2 corresponding to polymer (a-3) was prepared as follows.

MPC (11.7 g) and stearyl methacrylate (3.3 g) as C₂-C₂₀ AMA (MPC/C₂-C₂₀ AMA=80/20 (molar ratio)) were weighed into a glass flask for polymerization, ethanol (85.0 g) was added to dissolve same, and AIBN (0.06 g) was added to the obtained solution. The inside of the flask was sufficiently replaced with nitrogen, and polymerization was performed by heating at 60°C for 6 hr while stirring. Thereafter, in the same manner as in Synthetic Example 1, a random copolymer (hereinafter to be indicated as "polymer 3-2" in the present specification) was obtained. The weight average molecular weight of polymer 3-2 was 43,000 based on polyethylene glycol as measured by GPC under the below-mentioned conditions.

### [Synthetic Example 4]

Polymer 4 corresponding to polymer (a-4) was prepared as follows.

MPC (8.4 g), n-butyl methacrylate (2.1 g) as C₂-C₈ AMA, and glycerol monomethacrylate (4.5 g) as HO-AMA (MPC/C₂-C₂₀ AMA/HO-AMA=40/40/20 (molar ratio)) were weighed into a glass flask for polymerization, purified water (42.5 g) and ethanol (42.5 g) were added to dissolve same, and AIBN (0.15 g) was added to the obtained solution. Thereafter, in the same manner as in Synthetic Example 1, a random copolymer (hereinafter to be indicated as "polymer 4" in the present specification) was obtained. The weight average molecular weight of polymer 4 was 22,000 based on polyethylene glycol as measured by GPC under the below-mentioned conditions.

### [Synthetic Example 5]

Polymer 5 corresponding to polymer (a-5) was prepared as follows.

MPC (25.3 g) and methoxypolyethylene glycol methacrylate (number average molecular weight: about 500) (4.7 g) as PEG-MA (MPC/PEG-MA-90/10 (molar ratio)) were weighed into a glass flask for polymerization, purified water (70.0 g) was added to dissolve same, and AIBN (0.23 g) was added to the obtained solution. Thereafter, in the same manner as in Synthetic Example 1, a random copolymer (hereinafter to be indicated as "polymer 5") was obtained. The weight average molecular weight of polymer 5 was 501,000 based on polyethylene glycol as measured by GPC under the below-mentioned conditions.

### [GPC measurement]

GPC measurement of the polymers 1 to 5 obtained in Synthetic Examples 1 to 5 was performed under the following conditions.
GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 µL

The monomers used in Synthetic Examples 1 to 5 and molar ratios thereof, and weight average molecular weights of the obtained polymers are shown in Table 1.

**[Table 1]**

| | monomer 1 | monomer 2 | monomer 3 | molar ratio of monomer | weight average molecular weight |
|---|---|---|---|---|---|
| polymer 1 | MPC | - | - | 100/0/0 | 1,030,000 |
| polymer 2 | MPC | methacrylic acid | - | 30/70/0 | 680,000 |
| polymer 3-1 | MPC | n-butyl methacrylate | - | 80/20/0 | 600,000 |
| polymer 3-2 | MPC | stearyl methacrylate | - | 80/20/0 | 43,000 |
| polymer 4 | MPC | n-butyl methacrylate | glycerol monomethacrylate | 40/40/20 | 22,000 |
| polymer 5 | MPC | methoxypolyethylene glycol methacrylate | - | 90/10/0 | 501,000 |

| | | | | | |
|---|---|---|---|---|---|
| molar ratio of monomer = monomer 1/monomer 2/monomer 3 | | | | | |

### [Example 1-1 to Example 1-7 and Comparative Example 1-1 to Comparative Example 1-3]

Examples and Comparative Examples are shown in which the amplification target nucleic acid is a single-stranded RNA and the nucleic acid amplification method is an RT-PCR method.

### [Nucleic acid amplification-promoting agent]

Each polymer used in Example 1-1 to Example 1-7 and each additive used in Comparative Example 1-1 to Comparative Example 1-3 were used in the below-mentioned reaction compositions at the final concentrations shown in Tables 4 and 5. In each of Comparative Example 1-1 to Comparative Example 1-3, polyethylene glycol 6000 (hereinafter indicated as "PEG6000"), bovine serum albumin (hereinafter indicated as "BSA", manufactured by Sigma-Aldrich), and dimethyl sulfoxide (hereinafter indicated as "DMSO") were used. Control 1 is a control test not using the polymer of the present invention or comparison target additives but using nuclease-free sterilized water instead (hereinafter indicated as "PW").

### [Preparation of reaction composition]

Using components of a commercially available RT-PCR kit "SARS-CoV-2 RT-q PCR Detection Kit" (manufactured by FUJIFILM Wako Pure Chemical Corporation) and PW (manufactured by Nippon Gene Co., Ltd.), the reaction composition shown in Table 2 was prepared. The preparation was performed on ice, the nucleic acid amplification-promoting agent, reaction reagents, and sample were added in this order, and the below-mentioned nucleic acid amplification reaction was performed immediately.

**[Table 2]**

| component name | | added amount |
|---|---|---|
| nucleic acid amplification-promoting agent^{*1} | | given amount |
| reaction reagents | 5x Reaction Buffer^{*2} | 4 µL |
| | 2 mM dNTPs^{*2} | 4 µL |
| | 50 mM Manganese (II) Acetate^{*2} | 1 µL |
| | Hot Start Reverse Transcription DNA Polymerase^{*2} | 0.25 µL |
| | Fw Primer NIID_2019-nCOV_N_F2 (10 µM)^{*2} | 1 µL |
| | Rv Primer NIID_2019-nCOV_N_R2 (10 µM)^{*2} | 1.4 µL |
| | PW | rest |
| sample | Positive Control RNA, N set No.2 (40 copy/µL)^{*2} | 5 µL |
| total volume | | 20 µL |

| | | |
|---|---|---|
| *1: Polymer of each Example or additive of each Comparative Example shown in Tables 4 and 5 were added to the final concentrations described in Tables 4 and 5. *2: Component of SARS-CoV-2 RT-q PCR Detection Kit | | |

### [Nucleic acid amplification reaction]

The aforementioned reaction composition was dispensed to a PCR plate (MicroAmp^{™} Fast Optical 96-well Reaction Plate with Barcode, 0.1 mL, manufactured by AppliedBioscience), plate-sealed, this PCR plate was set on a PCR thermal cycler (manufactured by AppliedBioscience), and the RT-PCR method was performed using the temperature program shown in Table 3.

**[Table 3]**

| step # | temperature | time | cycle number |
|---|---|---|---|
| 1 | 90°C | 30 sec | |
| 2 | 60°C | 5 min | |
| 3 | 95°C | 1 min | |
| 4 | 95°C | 3 sec | 60 cycles |
| 5 | 60°C | 20 sec | |
| 6 | 4°C | ∞ | |

### [Quantification of nucleic acid]

Each nucleic acid amplification product obtained by the above procedure was quantified by the following procedure.

First, 5x Loading Buffer (manufactured by Nippon Gene Co., Ltd.) (4 µL) was added to the obtained nucleic acid amplification product (20 µL) and used as an electrophoresis sample.

On the other hand, purified agarose (trade name "Agarose21", manufactured by Nippon Gene Co., Ltd.) was suspended at 3 w/v% in 0.5x TBE buffer, heated and dissolved using a microwave oven, poured into a mold equipped with a comb, and allowed to cool and solidify to prepare an agarose gel for electrophoresis.

The above-mentioned electrophoresis sample was applied at 4 pL/well to the above-mentioned agarose gel for electrophoresis. A DNA marker (trade name "Gene Ladder 100", manufactured by Nippon Gene Co., Ltd.) was also applied at 4 µL/well.

Using 0.5x TBE buffer as an electrophoresis buffer, electrophoresis was performed for about 30 min by applying a voltage of 100V.

Thereafter, the gel was taken out, immersed in a staining solution prepared by diluting a nucleic acid staining reagent (trade name "Midori Green Advance", manufactured by Nippon Genetics Co., Ltd.) 10,000 times with 0.5x TBE buffer, and shaken gently for 30 min to stain the nucleic acid.

Thereafter, the gel was taken out, immersed in ultrapure water, and gently shaken for 15 min to remove excess nucleic acid staining reagent.

Thereafter, the gel was set in a gel imager (ChemiDocXRS+, manufactured by Bio-Rad) and the gel was photographed under irradiation with UV light for transmission. Using the image analysis software attached to the device described above, the staining intensity of the band of the target nucleic acid amplification product (size: 158 bp) was quantified, and the amount of amplified nucleic acid was calculated as a relative value with the measured value of control 1 as 100. The results (average value of two tests) are shown in Tables 4 and 5.

**[Table 4]**

| | control 1 | Example 1-1 | Example 1-2 | Example 1-3 | Example 1-4 | Example 1-5 |
|---|---|---|---|---|---|---|
| polymer or additive used | - | polymer 1 | polymer 2 | polymer 3-1 | polymer 3-2 | polymer 4 |
| final concentration (w/v%) of polymer or additive in reaction composition | - | 0.05 | 0.05 | 0.05 | 0.05 | 0.05 |
| amount of amplified nucleic acid | 100 | 377 | 526 | 488 | 606 | 515 |

**[Table 5]**

| | control 1 | Example 1-6 | Example 1-7 | Comparative Example 1-1 | Comparative Example 1-2 | Comparative Example 1-3 |
|---|---|---|---|---|---|---|
| polymer or additive used | - | polymer 1 | polymer 5 | PEG 6000 | BSA | DMSO |
| final concentration (w/v%) of polymer or additive in reaction composition | - | 0.1 | 0.1 | 0.1 | 0.1 | 2.0 |
| amount of amplified nucleic acid | 100 | 263 | 288 | 115 | 131 | 74 |

As shown in Tables 4 and 5, when the nucleic acid amplification-promoting agent of the present invention (i.e., polymers 1 to 5) was used, the amount of the nucleic acid amplification product increased about 2.6 to 6.1 times as compared with control 1. On the other hand, when each additive of Comparative Example was used, the amount of the nucleic acid amplification product was about 0.7 to 1.3 times as compared with that of the control 1. From these results, it can be said that the nucleic acid amplification-promoting agent of the present invention can favorably promote RNA amplification reaction.

### [Example 2]

As a more generalized example, an example is shown in which the amplification target nucleic acid is a double-stranded DNA and the nucleic acid amplification method is a typical PCR method.

### [Nucleic acid amplification-promoting agent]

Polymer 1 used in Example 2 was contained in the below-mentioned reaction composition at the final concentration shown in Table 8 and used. Control 2 is a control test using PW instead of polymer 1.

### [Preparation of reaction composition]

The reaction compositions shown in Table 6 was prepared. The preparation was performed on ice, the nucleic acid amplification-promoting agent, reaction reagents, and sample were added in this order, and the below-mentioned nucleic acid amplification reaction was performed immediately.

**[Table 6]**

| component name | | added amount |
|---|---|---|
| nucleic acid amplification-promoting agent (polymer 1)^{*1} | | given amount |
| reaction reagents | 10x PCR Buffer^{*2} | 2 µL |
| | 25 mM MgSO₄^{*2} | 1.2 µL |
| | 2 mM dNTPs^{*2} | 2 µL |
| | Primer Fw (3 µM)^{*3} | 2 µL |
| | Primer Rv (3 µM)^{*4} | 2 µL |
| | KOD-plus- (1 U/µL) | 0.4 µL |
| | PW | rest |
| sample | Lambda DNA (10 ng/µL)^{*5} | 5 µL |
| total volume | | 20 µL |

| | | |
|---|---|---|
| *1: Polymer 1 was added to the final concentration described in Table 8. *2: component of KOD-plus-Ver.2 *3: 5'-CAGCCGCGCTGGATGAACTGATACC-3' (sequence 1) (manufactured by Nippon Gene Co., Ltd.) *4: 5'-AGCGGAGACGGGCAATCAGTTCATC-3' (sequence 2) (manufactured by Nippon Gene Co., Ltd.) *5: manufactured by Nippon Gene Co., Ltd. | | |

### [Nucleic acid amplification reaction]

The aforementioned reaction composition was dispensed to a 0.1 mL 8 PCR tube (manufactured by FUKAE KASEI CO., LTD.), a flat cap was placed, the sample was set in a PCR thermal cycler (manufactured by AppliedBioscience), and a typical PCR method was performed with the temperature program shown in Table 7.

**[Table 7]**

| step # | temperature | time | cycle number |
|---|---|---|---|
| 1 | 94°C | 2 min | |
| 2 | 98°C | 10 sec | 40 cycles |
| 3 | 60°C | 30 sec | |
| 4 | 68°C | 6 min 20 sec | |
| 5 | 4°C | ∞ | |

### [Quantification of nucleic acid]

Each nucleic acid amplification product obtained by the above procedure was quantified by the following procedure.

First, 5x Loading Buffer (manufactured by Nippon Gene Co., Ltd.) (4 µL) was added to the obtained nucleic acid amplification product (20 µL) and used as an electrophoresis sample.

On the other hand, purified agarose (trade name "AgaroseS", manufactured by Nippon Gene Co., Ltd.) was suspended at 1 w/v% in 1x TAE buffer, heated and dissolved using a microwave oven, poured into a mold equipped with a comb, and allowed to cool and solidify to prepare an agarose gel for electrophoresis.

The above-mentioned electrophoresis sample was applied at 4 pL/well to the above-mentioned agarose gel for electrophoresis. A DNA marker (trade name "Gene Ladder Wide 1", manufactured by Nippon Gene Co., Ltd.) was also applied at 4 µL/well.

Using 1x TAE buffer as an electrophoresis buffer, electrophoresis was performed for about 30 min by applying a voltage of 100V.

Thereafter, the gel was taken out, immersed in a staining solution prepared by diluting a nucleic acid staining reagent (trade name "Midori Green Advance", manufactured by Nippon Genetics Co., Ltd.) 10,000 times with 1x TAE buffer, and shaken gently for 30 min to stain the nucleic acid.

Thereafter, the gel was taken out, immersed in ultrapure water, and gently shaken for 15 min to remove excess nucleic acid staining reagent.

Thereafter, the gel was set in a gel imager (FAS-Digi Compact, manufactured by Nippon Genetics Co., Ltd.) and the gel was photographed under irradiation with BlueGreen LED. A 16-bit TIFF image was output from the imaging device, the staining intensity of the band of the target nucleic acid amplification product (size: 6.4 kbp) was quantified using an image analysis software (ImageJ, National Institutes of Health), and the amount of amplified nucleic acid was shown as a relative value with the measured value of control 2 as 100. Table 8 shows the results of three times of test, and also indicates the p-value determined by Student's t-test (two-tailed test).

**[Table 8]**

| | | control 2 | Example 2 |
|---|---|---|---|
| polymer used | | - | polymer 1 |
| final concentration (w/v%) of polymer in reaction composition | | - | 0.05 |
| amount of amplified nucleic acid | 1 | 91 | 134 |
| | 2 | 98 | 132 |
| | 3 | 111 | 130 |
| | average | 100 | 132 |
| | standard deviation | 9.9 | 2.1 |
| p value | | 0.03 | |

As shown in Table 8, when the nucleic acid amplification-promoting agent of the present invention (i.e., polymer 1) was used, the amount of the nucleic acid amplification product significantly increased at the 5% level as compared with control 2. From this result, it can be said that the nucleic acid amplification-promoting agent of the present invention can favorably promote DNA amplification reactions.

From the above Examples, it can be said that the nucleic acid amplification-promoting agent of the present invention can favorably promote DNA amplification reactions, whether the amplification target is RNA or DNA.

### [Industrial Applicability]

Using the nucleic acid amplification-promoting agent of the present invention, the amount of nucleic acid amplification products obtained by the nucleic acid amplification method (particularly, PCR method) can be increased. Therefore, the nucleic acid amplification-promoting agent of the present invention can be preferably used to improve the yield of a desired nucleic acid by a nucleic acid amplification method, to improve the performance of tests by a nucleic acid amplification method, to improve the performance of sequencing by a nucleic acid amplification method, and the like.

This application is based on a patent application No. 2021-161241 filed in Japan, the contents of which are incorporated in full herein.

## Claims

1. A nucleic acid amplification-promoting agent consisting of a polymer comprising a constitutional unit derived from a monomer having a phosphorylcholine group.

2. The nucleic acid amplification-promoting agent according to claim 1, wherein the polymer comprises one or more polymers selected from the following group a:
[group a]
(a-1) a polymer comprising a constitutional unit derived from 2-(meth)acryloyloxyethylphosphorylcholine alone,
(a-2) a copolymer comprising a constitutional unit derived from 2-(meth)acryloyloxyethylphosphorylcholine and a constitutional unit derived from (meth)acrylic acid,
(a-3) a copolymer comprising a constitutional unit derived from 2-(meth)acryloyloxyethylphosphorylcholine and a constitutional unit derived from a C₂-C₂₀ alkyl (meth)acrylate,
(a-4) a copolymer comprising a constitutional unit derived from 2-(meth)acryloyloxyethylphosphorylcholine, a constitutional unit derived from a C₂-C₈ alkyl (meth)acrylate, and a constitutional unit derived from a C₂-C₈ alkyl (meth)acrylate having two or more hydroxy groups as substituents, and
(a-5) a copolymer comprising a constitutional unit derived from 2-(meth)acryloyloxyethylphosphorylcholine and a constitutional unit derived from a polyethylene glycol (meth)acrylate.

3. A test method using the nucleic acid amplification-promoting agent according to claim 1 or 2.
